# EUROPÄISCHE PATENTSCHRIFT

(11) **EP 3 224 619 B1**
(45) Veröffentlichungstag und Bekanntmachung des Hinweises auf die Patenterteilung: **02.01.2019**
(21) Anmeldenummer: 15832727.0
(22) Anmeldetag: 27.11.2015
(51) Int. Cl.: G01N 33/50, G01N 33/569

(54) **VERFAHREN ZUM NACHWEIS ANTIGEN-SPEZIFISCHER IMMUNZELLEN IN EXTRASANGUINEN FLÜSSIGKEITEN**
NEW METHOD FOR THE IMPROVED DETECTION OF ANTIGEN-SPECIFIC IMMUNE CELLS IN EXTRASANGUINE FLUIDS
PROCÉDÉ POUR LA DÉTECTION DE CELLULES IMMUNITAIRES SPÉCIFIQUES D'UN ANTIGÈNE DANS DES LIQUIDES EXTRA-SANGUINS

(30) Priorität: 29.11.2014 DE 102014018047
(43) Veröffentlichungstag der Anmeldung: 04.10.2017
(73) Patentinhaber: Genome Identification Diagnostics GmbH, 72479 Strassberg (DE)
(72) Erfinder: SCHMIDT, Tina, 66459 Kirkel (DE); SESTER, Martina, 66424 Homburg/Saar (DE); SCHUB, David, 66459 Kirkel (DE)
(74) Vertreter: Patentanwälte Ruff, Wilhelm, Beier, Dauster & Partner mbB
(86) Internationale Anmeldenummer: PCT/DE2015/000569
(87) Internationale Veröffentlichungsnummer: WO 2016/082819

(56) Entgegenhaltungen:
- WO-A1-97/45735
- WO-A1-2014/083201
- URBAN SESTER ET AL: "Rapid Identification of Preformed Alloreactive T Cells for Use in a Clinical Setting", TRANSPLANTATION, Bd. 78, Nr. 4, 1. August 2004 (2004-08-01) , Seiten 607-614, XP055058763, ISSN: 0041-1337, DOI: 10.1097/01.TP.0000131949.59284.4D
- Anil A Panackal ET AL: "Paradoxical Immune Responses in Non-HIV Cryptococcal Meningitis", Plos pathogens, 28. Mai 2015 (2015-05-28), Seiten 1-27, XP055268598, Gefunden im Internet: URL:http://journals.plos.org/plospathogens /article/asset?id=10.1371/journal.ppat.100 4884.PDF [gefunden am 2016-04-26]
- MARTINA SESTER ET AL: "The ABC of virus-specific T-cell immunity in solid organ transplantation", AMERICAN JOURNAL OF TRANSPLANTATION, 1. Dezember 2015 (2015-12-01), Seiten n/a-n/a, XP055268442, DK ISSN: 1600-6135, DOI: 10.1111/ajt.13684
- D. Schub ET AL: "Altered Phenotype and Functionality of Varicella Zoster Virus-Specific Cellular Immunity in Individuals With Active Infection", JOURNAL OF INFECTIOUS DISEASES. JID, vol. 211, no. 4, 1 September 2014 (2014-09-01), pages 600-612, XP055268383, US ISSN: 0022-1899, DOI: 10.1093/infdis/jiu500

## Beschreibung

Primärinfektionen und Reaktivierungen von bakteriellen und viralen Erregern können zu schwerwiegenden Komplikationen führen. Dabei zeigen verschiedene Erreger einen Tropismus für eine spezifische Organprovinz, und sind nur in geringen Mengen oder überhaupt nicht während der Erkrankungsphase im Blut nachweisbar. Als indirekter Nachweis für eine stattgefundene Auseinandersetzung des Organismus mit einem Erreger können zwar Erreger-spezifische Antikörper herangezogen werden, jedoch ergibt sich bei vielen Erkrankungen das Problem, dass eine diagnostische Lücke zwischen Infektion und dem nachweisbaren Anstieg von Antikörpertitern besteht. Zudem ist im Falle einer Reaktivierung oder Reinfektion die Wertigkeit eines einmalig bestimmten Antikörpertiters meist gering.

Als Paradebeispiel für diagnostisch schwer zugängliche Infektionen gelten die Entzündungen des zentralen Nervensystems (ZNS) die mit einer hohen Letalitätsrate bzw. schweren neurologischen Folgeschäden einhergehen können [1, 2]. Die derzeitige Diagnosestellung bakteriell oder viral bedingter Beeinträchtigungen des ZNS unterliegt zahlreichen Limitierungen, die im Folgenden beispielhaft für Infektionen mit Alphaherpesviren (Varizella-Zoster-Virus (VZV) und Herpes-Simplex-Virus 1 und 2 (HSV-1 und-2)) aufgeführt werden. Zum Einen tritt bei Beeinträchtigung des ZNS der Herpes-typische Hautausschlag - das offensichtlichste Anzeichen einer Alphaherpesvirus-Infektion/-Reaktivierung - oft erst verspätet oder gar nicht auf [3]. Zum Anderen führt der als Goldstandard verwendete Erregernachweis durch PCR im Liquor cerebrospinalis (Liquor), insbesondere bei VZV, zu einer gewissen Menge an falsch negativen Ergebnissen. Dies liegt an der begrenzten Zeitspanne, in der Viren überhaupt nachweisbar sind und kann ein adäquates Infektmonitoring unmöglich machen. Da also der anamnestische und der direkte Erregernachweis unzureichend sind, bieten sich Ansatzpunkte für eine immunbasierte Diagnostik. Erhöhte Virus-spezifische IgA-Titer werden bereits als Indiz für eine Virusbedingte ZNS-Erkrankung herangezogen, allerdings konnte beobachtet werden, dass es bei bestimmten ZNS-Erkrankungen, wie der multiplen Sklerose, auch unabhängig von einer Reaktivierung zu einem Anstieg dieser IgA-Titer kommt [4], d.h. auch in diesem Fall weist die Antikörper-Diagnostik Lücken auf.

Ein weiteres diagnostisches Mittel ist die Bestimmung der Zellzahl sowie der Zellmorphologie der sich im Liquor befindenden Zellen. Sowohl bei bakteriellen als auch bei viralen Infektionen des ZNS kommt es zum Anstieg der Zellzahl im Liquor (Pleozytose), wobei bakterielle Infektionen eher eine Anreicherung von Granulozyten und virale Infektionen eine Anreicherung von Lymphozyten aufweisen. Neuere Techniken, die bislang vorwiegend im Blut angewandt werden, ermöglichen darüber hinaus eine genauere Charakterisierung der Spezifität der Zellen. Die Quantifizierung Antigen-spezifischer Immunantworten erfolgt dabei anhand der spezifischen Zytokininduktion von T-Lymphozyten nach Stimulation [5, 6]. Am Beispiel der durch M. *tuberculosis* verursachten Meningitis konnte bereits gezeigt werden, dass sich bei bakteriellen Infektionen die im Liquor vorhandenen Erreger-spezifischen T-Zellen direkt auf Basis der Interferon-γ-Expression nachweisen lassen [7]. Diese Anreicherung von Antigen-spezifischen T-Zellen am Ort einer Infektion ist ein generelles immunologisches Prinzip. Entsprechend findet sich z.B. bei Patienten mit Lungentuberkulose auch eine Anreicherung Erreger-spezifischer T-Zellen in der bronchoalveolären Lavage [8,9]. Aber auch viele Autoimmunantworten (z.B. bei Multipler Sklerose) oder unerwünschte Immunantworten gegen übertragene Transplantate gehen mit einer Organ-spezifischen Anreicherung von gegen das jeweilige Antigen gerichteten Lymphozyten einher. Die Analyse dieser Zellen bietet somit die Möglichkeit, die spezifische Immunantwort nicht nur systemisch, sondern auch direkt am Ort des Infekt- bzw. Entzündungsgeschehens zu untersuchen. Somit könnte z.B. eine T-Zell-basierte Diagnostik von Infektionserkrankungen aus extrasanguinen Flüssigkeiten neben der Identifizierung des infektionsauslösenden Erregers einen wichtigen Beitrag zum Therapie-Monitoring leisten [10,11]. Die Charakterisierung der Antigen-spezifischen T-Zell-Antwort aus Blut und extrasanguinen Flüssigkeiten erfordert jedoch stets die Durchführung von zwei separaten Stimulationsreaktionen. Zudem ist gerade der Nachweis von spezifischen T-Zellen in extrasanguinen Flüssigkeiten aufgrund des limitierten Probenumfangs (Liquor steht z.B. im Vergleich zum Blut in nur geringen Volumina zur Verfügung) oder aufgrund der geringen Zellzahl in den gewonnenen Proben technisch oft erschwert oder nicht möglich.

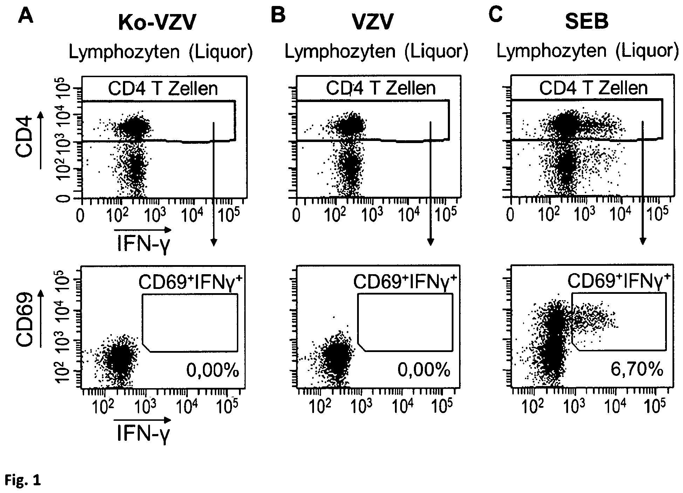

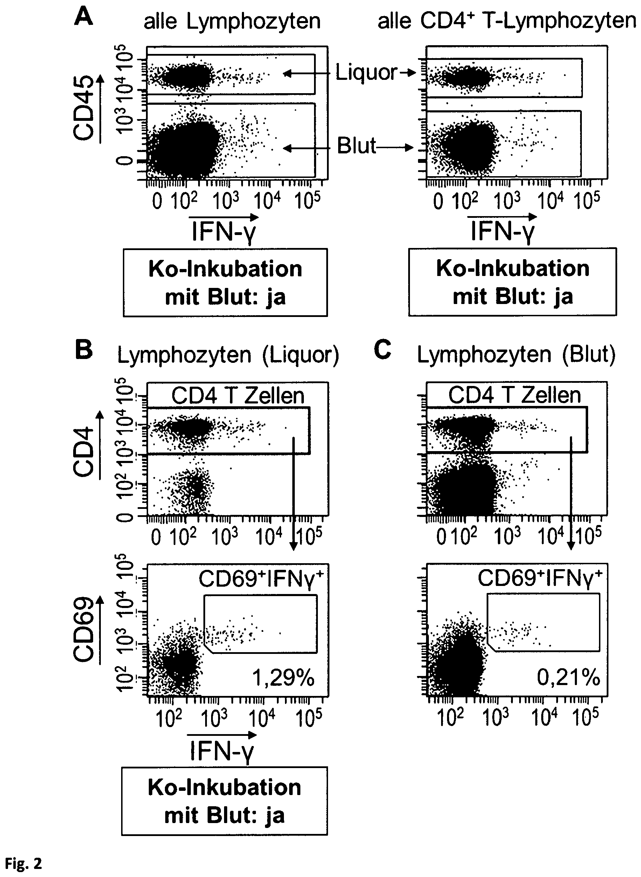

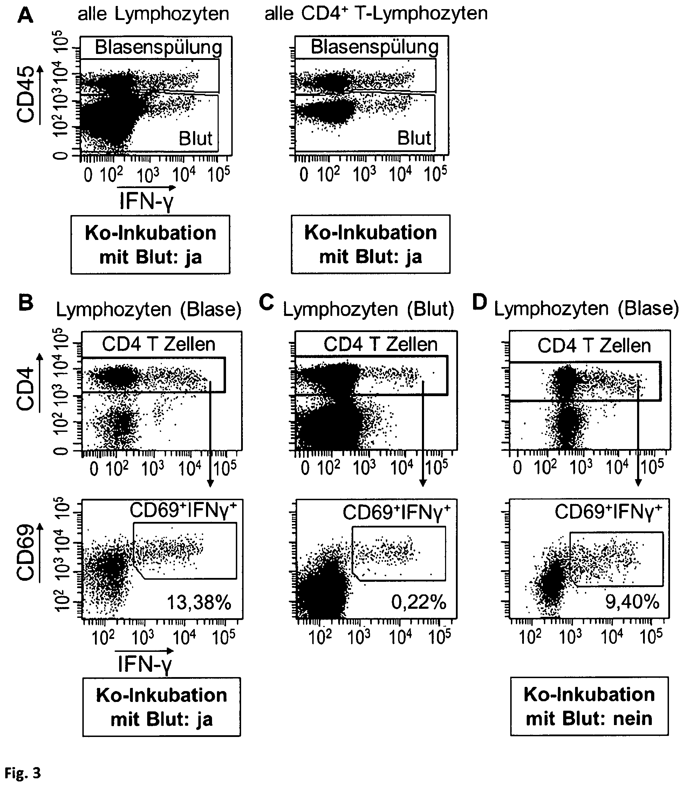

Am Beispiel unserer Ergebnisse im Rahmen der Entwicklung eines T-Zell-basierten diagnostischen Verfahrens für VZV-bedingte ZNS-Infektionen konnten wir darüber hinaus zeigen, dass ein zusätzlicher limitierender Faktor die geringe Anzahl an Antigenpräsentierenden Zellen (APCs) in extrasanguinen Flüssigkeiten (in diesem Falle im Liquor) sein könnte, die eine Antigen-abhängige Aktivierung der potentiell vorhandenen Virusspezifischen T-Zellen zusätzlich verhindert (Fig. 1). Um diese limitierenden Schritte zu umgehen, werden in einem neuen Assay extrasanguine und Vollblut-Proben der gleichen Person vermischt und anschließend simultan mit dem interessierenden Antigen stimuliert. Dies führt dazu, dass die im Blut befindlichen APCs sowohl Antigen-spezifische T-Zellen aus dem Blut als auch aus der extrasanguinen Flüssigkeit aktivieren und eine spezifische Zytokininduktion hervorrufen, anhand derer die Antigen-spezifischen T-Zellen identifiziert werden können. Das besondere Merkmal unseres neuen Analyseverfahrens ist dabei, dass alle extrasanguinen Zellen mit einem Fluorochrom-gekoppelten CD45-Antikörper angefärbt werden, bevor sie mit den Blutzellen vermengt werden. Dies führt dazu, dass bei der abschließenden Analyse T-Zellen aus dem Blut und T-Zellen aus der extrasanguinen Flüssigkeit aufgrund des Fehlens bzw. des Vorhandenseins dieses Fluoreszenzsignals diskriminiert werden können, obwohl die Zellen während der Verarbeitung miteinander vermischt waren (Fig. 2). Die Allgemeingültigkeit dieses Prinzips konnte bei der Untersuchung von spezifisch gegen Bestandteile des Bacille Calmette Guerin "BCG" (PPD) gerichteten T-Zellen in Blasenspülflüssigkeit bei Patienten mit BCG-Infektionen der Blase bestätigt werden (Fig. 3).

Unser neues Testverfahren ermöglicht also eine sensitivere quantitative und qualitative Analyse Antigen-spezifischer T-Zellen aus extrasanguinen Flüssigkeiten, die als diagnostisches Nachweisverfahren und zum Monitoring des Behandlungserfolgs Erreger-spezifischer, aber auch durch gegen Autoantigene oder Alloantigene gerichtete T-Zellen bedingte Organ-Erkrankungen Anwendung finden könnte. Zudem erlaubt diese technische Erfindung die Möglichkeit zur simultanen Quantifizierung und Analyse Antigen-spezifischer T-Zellen aus extrasanguiner Flüssigkeit und Blut und bietet somit die Möglichkeit, in einer einzigen Stimulationsreaktion Verschiebungen Antigen-spezifischer T-Zellen in extrasanguinen Flüssigkeiten im Vergleich zum Blut zu ermitteln. Über die hier dargestellten Beispiele hinaus könnte dieses Testverfahren auf alle anderen Erreger - beispielsweise mit ZNS-Beteiligung (z.B. HSV-1 und 2, Borrelien, FSME-Virus) - übertragen werden und dort einen entscheidenden Beitrag zur Diagnostik leisten. Das methodische Prinzip kann außerdem zur Identifizierung Erreger-spezifischer T-Zellen aus anderen Körperflüssigkeiten herangezogen werden (z.B. Bronchoalveoläre Lavage, Pleuraflüssigkeit, Blasenspülflüssigkeit). Des Weiteren bietet der methodische Ansatz ebenfalls eine Ausweitung der Analyse von T-Zellen mit Spezifität gegen andere, nicht-infektiöse Antigene und kann folglich auch bei Patienten mit Autoimmunerkrankungen, nach Organtransplantation oder bei Tumoren angewandt werden.

### Schlagwörter

zelluläre Immunität, virale Infektion, extrasanguine Flüssigkeiten, Vollblut, Immundiagnostik

### Erläuterungen zu den Zeichnungen

**Fig. 1****: Keine Detektion Erreger-spezifischer T-Zellen bei Stimulation von Liquorzellen.** In Abwesenheit von Blut können nach Stimulation von CD4 T-Zellen im Liquor weder mit dem Kontroll-Antigen (Ko-VZV; Negativkontrolle) (A) noch mit dem Varizella-Zoster-Virus-Antigen (VZV) (B) aktivierte (CD69⁺IFN-γ⁺) CD4 T-Zellen (jeweils unten) detektiert werden. Lediglich unter Verwendung eines polyklonalen Stimulus (SEB; Positivkontrolle) (C) ist anhand der doppelt CD69- sowie Interferon-y (IFN-γ)-positiven CD4 T-Zellen eine Aktivierung nachweisbar. Die Prozentzahlen beziehen sich jeweils auf die Frequenz aktivierter (CD69⁺IFN-γ⁺) CD4 T-Zellen.
**Fig. 2****: Simultane Analyse Erreger-spezifischer T-Zellen aus extrasanguiner Flüssigkeit (Liquor) und Blut.** Die Vorfärbung der Liquorzellen mit Fluorochrom-gekoppeltem CD45-Antikörper ermöglicht die Diskriminierung von Zellen aus Blut (CD45-negativ) und Liquor (CD45-positiv). (A) Dargestellt ist die Trennung der beiden Populationen auf Ebene der Lymphozyten (links) bzw. CD4 T-Zellen (rechts). (B) Interferon-y (IFN-γ)-Expression VZV-spezifischer CD4 T-Zellen aus Liquor nach Stimulation in Anwesenheit von Blut derselben Person ist nachweisbar, während diese in Abwesenheit von Blut nicht nachweisbar ist (siehe Fig. 1B). (C) Simultane Analyse der IFN-γ-Expression VZV-spezifischer CD4 T-Zellen aus Blut. Die Prozentzahlen beziehen sich jeweils auf die Frequenz aktivierter (CD69⁺IFN-γ⁺) CD4 T-Zellen.
**Fig. 3****: Simultane Analyse Erreger-spezifischer T-Zellen aus extrasanguiner Flüssigkeit (Blasenspülflüssigkeit) und Blut.** Die Vorfärbung der Zellen aus der Blasenspülung mit Fluorochrom-gekoppeltem CD45-Antikörper ermöglicht die Diskriminierung von Zellen aus Blut (CD45-negativ) und Blasenspülung (CD45-positiv). (A) Dargestellt ist die Trennung der beiden Populationen auf Ebene der Lymphozyten (links) bzw. CD4 T-Zellen (rechts). (B) Interferon-γ (IFN-γ)-Expression PPD-spezifischer CD4 T-Zellen aus Blasenspülflüssigkeit (Blase) nach Stimulation in Anwesenheit von Blut derselben Person ist nachweisbar - und zwar in einer um über 40% gesteigerten Frequenz gegenüber der Stimulation ohne Blut (vgl. (D)). (C) Simultane Analyse der Interferon-γ-Expression PPD-spezifischer CD4 T-Zellen aus Blut. (D) Analyse der Interferon-γ-Expression PPD-spezifischer CD4 T-Zellen aus Blasenspülflüssigkeit ohne Ko-Inkubation mit Blut. Die Prozentzahlen beziehen sich jeweils auf die Frequenz aktivierter (CD69⁺IFN-γ⁺) CD4 T-Zellen.

### Zitierte Nichtpatentliteratur

1. Tyler, K.L., Herpes simplex virus infections of the central nervous system: encephalitis and meningitis, including Mollaret's. Herpes, 2004. 11 Suppl 2: p. 57A-64A.
2. Nagel, M.A. and D. Gilden, Complications of Varicella Zoster Virus Reactivation. Curr Treat Options Neurol, 2013.
3. Gilden, D., et al., Neurological disease produced by varicella zoster virus reactivation without rash. Curr Top Microbiol Immunol, 2010. 342: p. 243-53.
4. Reiber, H., S. Ungefehr, and C. Jacobi, The intrathecal, polyspecific and oligoclonal immune response in multiple sclerosis. Mult Scler, 1998. 4(3): p. 111-7.
5. Letsch, A. and C. Scheibenbogen, Quantification and characterization of specific T-cells by antigen-specific cytokine production using ELISPOT assay or intracellular cytokine staining. Methods, 2003. 31(2): p. 143-9.
6. Schmidt, T. and M. Sester, Detection of antigen-specific T cells based on intracellular cytokine staining using flow-cytometry. Methods Mol Biol, 2013. 1064: p. 267-74.
7. Thomas, M.M., et al., Rapid diagnosis of Mycobacterium tuberculosis meningitis by enumeration of cerebrospinal fluid antigen-specific T-cells. Int J Tuberc Lung Dis, 2008. 12(6): p. 651-7.
8. Jafari, C., et al., Rapid diagnosis of smear-negative tuberculosis by bronchoalveolar lavage enzyme-linked immunospot. Am J Respir Crit Care Med, 2006. 174(9): p. 1048-54.
9. Jafari, C., et al., Impact of a Mycobacterium tuberculosis-specific interferon-gamma release assay in bronchoalveolar lavage fluid for a rapid diagnosis of tuberculosis. J Intern Med, 2011. 270(3): p. 254-62.
10. Malavige, G.N., et al., Rapid effector function of varicella-zoster virus glycoprotein I-specific CD4+ T cells many decades after primary infection. J Infect Dis, 2007. 195(5): p. 660-4
11. Schub, D. et al, Altered phenotype and functionality of Varicella Zoster virus-specific cellular immunity in individuals with active infection, J Infect Dis, 2015, 211(4), p. 600-12; elektronisch veröffentlicht 01.09.2014.

## Patentansprüche

1. Verfahren zum verbesserten Nachweis Antigen-spezifischer Immunzellen in extrasanguinen Flüssigkeiten, bestehend aus folgenden Arbeitsschritten:
- Isolierung der vitalen Immunzellen aus einer extrasanguinen Flüssigkeit durch geeignete Wasch- und Zentrifugationsschritte, z.B. unter der Verwendung von physiologischer Kochsalzlösung als Waschpuffer und der Zentrifugation bei 200 bis 500 g,
- Bestimmung der Gesamtzellzahl, beispielsweise werden je Stimulationsansatz 10⁵ bis 1,5x10⁶ Zellen benötigt,
- Vorfärbung der isolierten Immunzellen mit einem Fluorochrom-gekoppelten Antikörper, welcher gegen auf den Zellen exprimierte Antigene, z.B. CD45, gerichtet ist oder mittels für die Färbung von vitalen Zellen geeigneten Fluorochromen, z.B. Carboxyfluorescein Diacetat Succinimidyl Ester, CFDA-SE,
- Entfernung ungebundener Antikörper oder Fluorochrome durch Waschschritte **gekennzeichnet durch**
- Vermischen der vorgefärbten Immunzellen mit heparinisiertem Vollblut derselben Person,
- Stimulierung der gemischten Proben mit dem interessierenden Antigen, mit oder ohne Zusatz von ko-stimulatorischen Antikörpern anti-CD49d und anti-CD28, anti-CD49d alleine oder anti-CD28 alleine,
- Inkubation für 30min bis 4h bei 35 bis 39°C und 3 bis 10% CO₂,
- Zugabe eines Sekretionsinhibitors, z.B. Brefeldin A, Monensin oder andere,
- Inkubation für 2 bis 12 Stunden bei 35 bis 39°C und 3 bis 10% CO₂,
- Lyse von Erythrozyten und Fixierung der Leukozyten,
- Waschschritte zur Isolierung der Leukozyten,
- Erhöhung der Zellmembrandurchlässigkeit durch Zugabe einer Zellmembran-Permeabilität-erhöhenden Substanz, z.B. Saponin,
- Inkubation mit spezifischen Fluorochrom-markierten Antikörpern gegen Aktivierungsmarker, z.B. anti-CD69, anti-IFN-γ, für mindestens 10min auf Eis oder bei Raumtemperatur oder bei 35 bis 39°C,
- Erneute Waschschritte zur Entfernung ungebundener Antikörper,
- Analyse der Zellen an einem Durchflusszytometer und Diskriminierung der Immunzellen aus extrasanguiner Flüssigkeit von denen aus dem Blut anhand des Fluoreszenzsignals, das zur Vorfärbung verwendet wurde, z.B. Fluorochrom des CD45-Antikörpers, CFSE, wobei die gegen den Stimulus reagierenden Zellen anhand der Positivität in Bezug auf die Aktivierungsmarker von den nicht reagierenden Zellen unterschieden werden.

2. Verfahren gemäß Patentanspruch 1, wobei ein Ansatz als Negativkontrolle durchgeführt wird, der bis auf die Zugaben von Antigen identisch ist zu besagtem Ansatz aus Patentanspruch 1 und bei dem statt des Antigens die Zugabe einer entsprechenden Menge eines physiologischen Puffers bzw. Lösungsmittels, z.B. PBS oder eines nicht-infizierten Zell-Lysates bzw. eines nicht immunstimulierenden Protein- oder Peptidgemisches zu den Immunzellen aus extrasanguiner Flüssigkeit und Blut erfolgt.

3. Verfahren gemäß Patentanspruch 2, wobei eine Positivkontrolle durchgeführt wird durch Zugabe eines polyklonalen Stimulus, z.B. *Staphylococcus aureus* Enterotoxin B, SEB, zu den Immunzellen aus extrasanguiner Flüssigkeit und Blut in einem dritten Ansatz, der ansonsten identisch ist zu besagtem ersten Ansatz.

4. Verfahren gemäß zumindest einem der Patentansprüche 1 bis 3, wobei das Verhältnis aus dem relativen Anteil der durch das Antigen aktivierten Immunzellen in extrasanguinen Flüssigkeiten zu dem relativen Anteil der durch das Antigen aktivierten Immunzellen im Blut ein Maß für die Krankheitsaktivität in dem Körperkompartiment darstellt, aus dem die extrasanguine Flüssigkeit gewonnen wurde.

5. Verfahren gemäß zumindest einem der Patentansprüche 1 bis 4, wobei extrasanguine Flüssigkeit und Blut menschlichen Ursprungs sind.

6. Verfahren gemäß zumindest einem der Patentansprüche 1 bis 5, wobei die vorgefärbten Immunzellen mit 100 µl bis 1500 µl heparinisiertem Vollblut je Stimulationsansatz vermischt werden.

## Claims

1. Method for improved detection of antigen-specific immune cells in extrasanguine fluids, comprising the following process steps:
- isolating of vital immune cells from an extrasanguine fluid by appropriate washing and centrifugation steps, e.g. using physiological saline solution as a wash buffer and centrifugation at 200 to 500 g,
- determining the total cell count, for example 10⁵ to 1.5x10⁶ cells are needed per stimulation batch,
- prestaining of the isolated immune cells using a fluorochrome-coupled antibody which is aimed at the antigens expressed on the cells, e.g. CD45, or using fluorochromes capable of staining vital cells, e.g. carboxyfluorescein diacetate succinimidyl ester, CFDA-SE,
- removing of unbound antibodies or fluorochromes by washing steps, **characterized by**
- mixing of the prestained immune cells with heparinized whole blood of the same individual patient,
- stimulating the mixed samples with the interesting antigen, with or without addition of co-stimulatory antibodies anti-CD49d and anti-CD28, anti-CD49d alone or anti-CD28 alone,
- incubation for 30min to 4h at 35 to 39°C and 3 to 10% CO₂,
- adding of a secretion inhibitor, e.g. brefeldin A, monensin or others,
- incubation for 2 to 12 hours at 35 to 39°C and 3 to 10% CO₂,
- lysis of erythrocytes and fixation of the leucocytes,
- washing steps for isolating the leucocytes,
- increasing cell membrane permeability by addition of a cell membrane permeability increasing substance, e.g. saponin,
- incubation with specific fluorochrome-marked antibodies against activation markers, e.g. anti-CD69, anti-IFN-γ, for at least 10min on ice or at room temperature or at 35 to 39°C,
- repeated washing steps for removing unbound antibodies,
- analysis of the cells on a flow cytometry device and discriminating the immune cells from extrasanguine fluid against those from blood based on the fluorescence signal which was used for the prestaining, e.g. fluorochrome of the CD45 antibody, CFSE, wherein the cells responding to the stimulus are distinguished from the non-responding cells based on the positivity in relation to the activation markers.

2. Method according to claim 1, wherein one batch is performed as a negative control, which is identical to said batch of claim 1 with the exception of the addition of antigen, and wherein instead of the antigen the addition of a corresponding amount of a physiological buffer or solvent, respectively, e.g. PBS or a not infected cell lysate or a not immune stimulating protein or peptide mixture to the immune cells from extrasanguine fluid and blood is performed.

3. Method according to claim 2, wherein a positive control is performed by addition of a polyclonal stimulus, e.g. *Staphylococcus aureaus* enterotoxin B, SEB, to the immune cells from extrasanguine fluid and blood in a third batch, which otherwise is identical to said first batch.

4. Method according to at least one of claims 1 to 3, wherein the ratio of relative proportion of the immune cells activated by the antigen in extrasanguine fluids to the relative proportion of the immune cells activated by the antigen in blood is a measure for the disease activity in the body compartment from which the extrasanguine fluid was taken.

5. Method according to at least one of claims 1 to 4, wherein extrasanguine fluid and blood are of human origin.

6. Method according to at least one of claims 1 to 5, wherein the prestained immune cells are mixed with 100 µl to 1500 µl of heparinized whole blood per stimulation batch.

## Revendications

1. Procédé de détection améliorée de cellules immunitaires spécifiques à un antigène dans des liquides extra-sanguins, constitué par les étapes suivantes :
- l'isolement des cellules immunitaires vitales à partir d'un liquide extra-sanguin par des étapes de lavage et de centrifugation appropriées, p. ex. en utilisant une solution physiologique de sel de cuisine en tant que tampon de lavage et une centrifugation à 200 à 500 g,
- la détermination du nombre de cellules total, 10⁵ à 1,5 x 10⁶ cellules étant par exemple nécessaires par préparation de stimulation,
- la pré-coloration des cellules immunitaires isolées avec un anticorps couplé à un fluorochrome, qui est dirigé contre des antigènes exprimés sur les cellules, p. ex. CD45, ou au moyen de fluorochromes appropriés pour la coloration de cellules vitales, p. ex. l'ester succinimidylique de diacétate de carboxyfluorescéine, CFDA-SE,
- l'élimination des anticorps ou fluorochromes non liés par des étapes de lavage, **caractérisé par**
- le mélange des cellules immunitaires pré-colorées avec du sang entier héparinisé de la même personne,
- la stimulation des échantillons mélangés avec l'antigène d'intérêt, avec ou sans ajout d'anticorps co-stimulateurs anti-CD49d et anti-CD28, anti-CD49d seuls ou anti-CD28 seuls,
- l'incubation pendant 30 minutes à 4 h à 35 à 39 °C et 3 à 10 % de CO₂,
- l'ajout d'un inhibiteur de sécrétion, p. ex. la bréfeldine A, la monensine ou d'autres,
- l'incubation pendant 2 à 12 heures à 35 à 39 °C et 3 à 10 % de CO₂,
- la lyse d'érythrocytes et la fixation des leucocytes,
- des étapes de lavage pour l'isolement des leucocytes,
- l'augmentation de la perméabilité de la membrane cellulaire par ajout d'une substance augmentant la perméabilité de la membrane cellulaire, p. ex. la saponine,
- l'incubation avec des anticorps marqués par des fluorochromes spécifiques contre des marqueurs d'activation, p. ex. anti-CD69, anti-IFN-γ, pendant au moins 10 minutes sur de la glace ou à température ambiante ou à 35 à 39 °C,
- de nouvelles étapes de lavage pour l'élimination des anticorps non liés,
- l'analyse des cellules sur un cytomètre à flux et la discrimination des cellules immunitaires du liquide extra-sanguin de celles du sang à l'aide du signal de fluorescence qui a été utilisé pour la pré-coloration, p. ex. un fluorochrome de l'anticorps CD45, CFSE, les cellules réagissant contre le stimulus étant différenciées des cellules ne réagissant pas à l'aide de la positivité vis-à-vis des marqueurs d'activation.

2. Procédé selon la revendication 1, dans lequel une préparation est réalisée en tant que témoin négatif, qui est identique à ladite préparation selon la revendication 1 à l'exception des ajouts d'antigène et pour laquelle l'ajout d'une quantité correspondante d'un tampon ou solvant physiologique, p. ex. le PBS ou un lysat cellulaire non infecté ou un mélange de protéines ou de peptides non immunostimulant, aux cellules immunitaires du liquide extra-sanguin et du sang a lieu au lieu de l'antigène.

3. Procédé selon la revendication 2, dans lequel un témoin positif est réalisé par ajout d'un stimulus polyclonal, p. ex. l'entérotoxine B de *Staphylococcus aureus*, SEB, aux cellules immunitaires du liquide extra-sanguin et du sang dans une troisième préparation, qui est sinon identique à ladite première préparation.

4. Procédé selon au moins l'une quelconque des revendications 1 à 3, dans lequel le rapport entre la proportion relative des cellules immunitaires activées par l'antigène dans les liquides extra-sanguins et la proportion relative des cellules immunitaires activées par l'antigène dans le sang constitue une mesure pour l'activité de la maladie dans le compartiment du corps à partir duquel le liquide extra-sanguin a été obtenu.

5. Procédé selon au moins l'une quelconque des revendications 1 à 4, dans lequel le liquide extra-sanguin et le sang sont d'origine humaine.

6. Procédé selon au moins l'une quelconque des revendications 1 à 5, dans lequel les cellules immunitaires pré-colorées sont mélangées avec 100 µl à 1 500 µl de sang entier héparinisé par préparation de stimulation.
